Europäisches Patentamt

European Patent Office

Office européen des brevets

(19)

(11) Veröffentlichungsnummer: **0 019 062**
**B1**

(12) **EUROPÄISCHE PATENTSCHRIFT**

(45) Veröffentlichungstag der Patentschrift: **29.06.83**

(51) Int. Cl.³: **A 61 B 17/18**

(21) Anmeldenummer: **80101615.5**

(22) Anmeldetag: **26.03.80**

(54) Cerclageschloss zur Verbindung der Enden eines Cerclagedrahtes bei Knochenbrüchen und Spannhalter zur Aufnahme des Schlosses.

(30) Priorität: **26.03.79 DE 2911748**

(43) Veröffentlichungstag der Anmeldung:
**26.11.80 Patentblatt 80/24**

(45) Bekanntmachung des Hinweises auf die Patenterteilung:
**29.06.83 Patentblatt 83/26**

(84) Benannte Vertragsstaaten:
**AT CH FR GB IT**

(56) Entgegenhaltungen:
**DE - C - 933 289**
**US - A - 1 252 260**
**US - A - 2 049 361**
**US - A - 3 469 573**

(73) Patentinhaber: **Dawidowski, Gerhard**
**Schützenstrasse 15**
**D-8170 Bad Tölz (DE)**

(72) Erfinder: **Dawidowski, Gerhard**
**Schützenstrasse 15**
**D-8170 Bad Tölz (DE)**

(74) Vertreter: **Vogeser, Werner, Dipl.-Ing.**
**Franz-Joseph-Strasse 39**
**D-8000 München 40 (DE)**

## Cerclageschloss zur Verbindung der Enden eines Cerclagedrahtes bei Knochenbrüchen und Spannhalter zur Aufnahme des Schlosses

Die Erfindung betrifft ein Cerclageschloß zur Verwendung bei Knochenbrüchen, das zur Verbindung der Drahtenden eines um einen Knochen gespannten Drahtes dient.

Die Anwendung der Drahtcerclage bei Knochenbrüchen ist bekannt und hat sich vom medizinischen Standpunkt auch bereits als erfolgreich erwiesen, sie hatte jedoch bisher bestimmte Einschränkungen, die durch die Festigkeit des Cerclagedrahtes bedingt sind.

Es ist bekannt, die beiden Drahtenden des Cerclagedrahtes zur Spannung zusammenzudrehen und dann abzukneifen Dabei entsteht jedoch ein Lockerungseffekt, da sich der Draht etwas aufdreht. Ein weiterer Lockerungseffekt entsteht dadurch, daß das stehengebliebene, zusammengedrehte Drahtstück umgebogen werden muß, um das Gewebe nicht zu beschädigen. Bei diesem Verfahren entspricht die Festigkeit des Drahtes dessen doppelter Biegefestigkeit.

Bei einem anderen bekannten Verfahren ist das eine Drahtende mit einer Öse versehen, durch die das andere Drahtende geführt wird. Die so entstehende Schlinge kann mit einem Gerät gespannt werden, an dem die Spannkraft, nicht jedoch die Fließgrenze, bei der der Draht reißt, angezeigt wird. Nach dem Spannen wird der Draht umgebogen und abgekniffen. Das umgebogene Drahtende hat dabei nur eine Spannung, die der einfachen Biegefestigkeit, und nicht mehr der vorher abgelesenen Spannkraft entspricht.

Der Erfindung liegt die Aufgabe zugrunde, ein Cerclageschloß zu schaffen, bei dem der Cerclagedraht definiert, d.h. ohne zu reißen, bis zu seiner Fließgrenze gespannt werden kann.

Gelöst wird diese Aufgabe demäß der Erfindung durch die im kennzeichnenden Teil des Anspruchs 1 angegebenen Merkmale. Zweckmäßige Ausgestaltungen der Erfindung, insbesondere ein Werkzeug zur Handhabung des Schlosses, ergeben sich aus den Unteransprüchen.

Durch die vorgeschlagene Ausbildung des Schlosses erreicht man, daß der Draht nur auf Zug beansprucht wird, da das eine Drahtende, das zum Spannen des Drahtes dient, an der Einspannstelle weder umgebogen noch geknickt wird.

Die Erfindung wird nachstehend anhand der Fig. 1 bis 4 beispielsweise erläutert. Es zeigt:

Fig. 1 teilweise im Schnitt eine perspektivische Darstellung des Schlosses,

Fig. 2 eine Aufsicht des Schlosses,

Fig. 3 eine Seitenansicht des Spannhalters, und

Fig. 4 eine Seitenansicht des Drehmomentschlüssels.

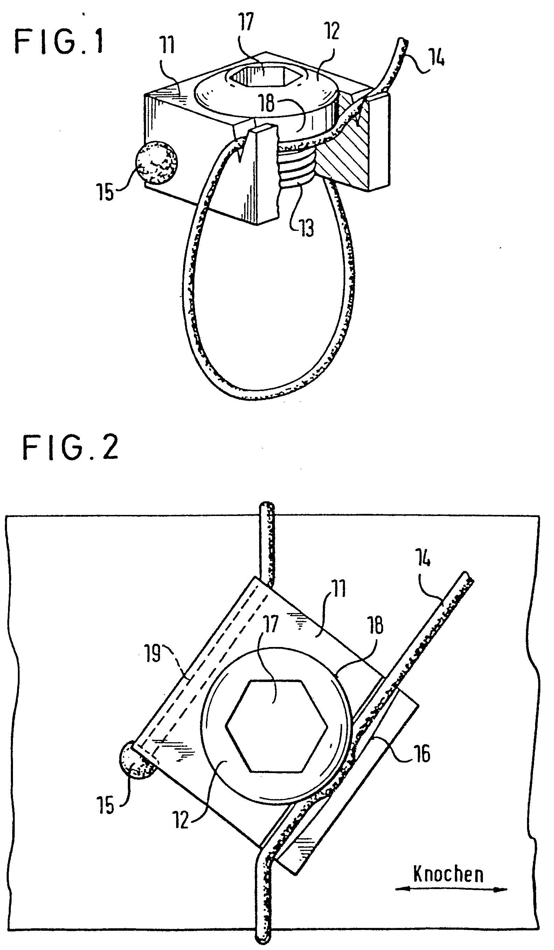

Fig. 1 zeigt perspektivisch und teilweise im Schnitt das Schloß 11, das aus einem rechteckigen Block mit den Abmessunen 5,5 × 5,5 × 3,5 mm besteht. Das Schloß 11 hat eine Gewindebohrung zur Aufnahme des Gewindeteils 13 einer Inbusschraube 12, sowie eine zylindrische Bohrung 18 zur Aufnahme des Kopfes der Schraube. Längs der einen Seite des Schlosses 11 verläuft eine Bohrung 19 zur Aufnahme des einen Endes eines Cerclagedrahts 14, der mit einer Kugel 15 am Drahtende am Schloß 11 anliegt. Die Bohrung 19 verläuft von dem Ende, am dem sich die Kugel 15 befindet von unten schräg nach oben.

An der der Bohrung 19 gegenüberliegenden Seite ist an der Oberseite des Schlosses 11 eine Keilnut 16 z.B. mittels eines Winkelfräsers mit einem Winkel von 120° gebildet, so daß die Keilnut 16 nach außen und unten gewölbt ist. Die Keilnut 16 ist so bemessen und angeordnet, daß der durchgeführte Draht 14 unter dem Kopf der Schraube 12 zu liegen kommt, den Gewindteil der Schraube aber nicht berührt. Im eingeführten Zustand drückt der Kopf der Schraube den Draht in die Keilnut, so daß dieser entsprechend der Form der Keilnut verformt wird, wie Fig. 1 zeigt.

Bei der Operation wird das Schloß 11, in das das eine Ende des Drahtes 14, das die Kugel 15 aufweist, eingeführt ist, in einen Spannhalter 20 eingesetzt. Der Spannhalter 20 hat ein Rohr 21 mit einer durchgehenden Bohrung 27, die am unteren Ende in eine Vierkantöffnung 25 zur Aufnahme des Schlosses 11 übergeht. Die eine Seite der Vierkantöffnung ist als Spannbacke ausgebildet und kann mittels eines Hebels 22, der durch eine Schraube 24 befestigt und schwenkbar gelagert ist, gegen das Schloß gedrückt werden. Hierzu wird eine Rändelmutter 23 am hinteren Ende des Hebels 22 derart gedreht, daß das hintere Ende des Hebels angehoben und damit das vordere gegen das Schloß gedrückt wird.

Nachdem das freie Ende des Drahtes 14 mit einem üblichen Instrument um den Knochen geführt worden ist, wird es in einem am unteren Ende des Rohres 21 ausgebildeten Führungstrichter 26 in die Keilnut 16 eingeführt und durchgeschoben. Die Kugel 15 kommt dabei in einer neben dem Führungstrichter 26 ausgebildeten Ausnehmung zu liegen.

Mittels einer Flachzange wird nun das freie Ende des Drahtes 14 bis zu einer Spannspindel 28 gezogen, die am oberen Ende des Rohrs 21 seitlich und schräg nach oben verlaufend in einer Hülse 29 drehbar und axial unverschiebbar gelagert ist. Die Spannspindel hat eine konische Nut 30, deren eine, die Spannung bewirkende Kante unter einem Winkel von 45° abgeschrägt ist. Der Draht 14 wird in der konischen Nut 30 festgeklemmt und durch Drehen der Spannspindel an deren Griff 31 gespannt. Bei diesem Vorgang wird der Spannhalter en einem etwa rechtwinklig zum Rohr 21 verlaufenden Griff 32 gehalten.

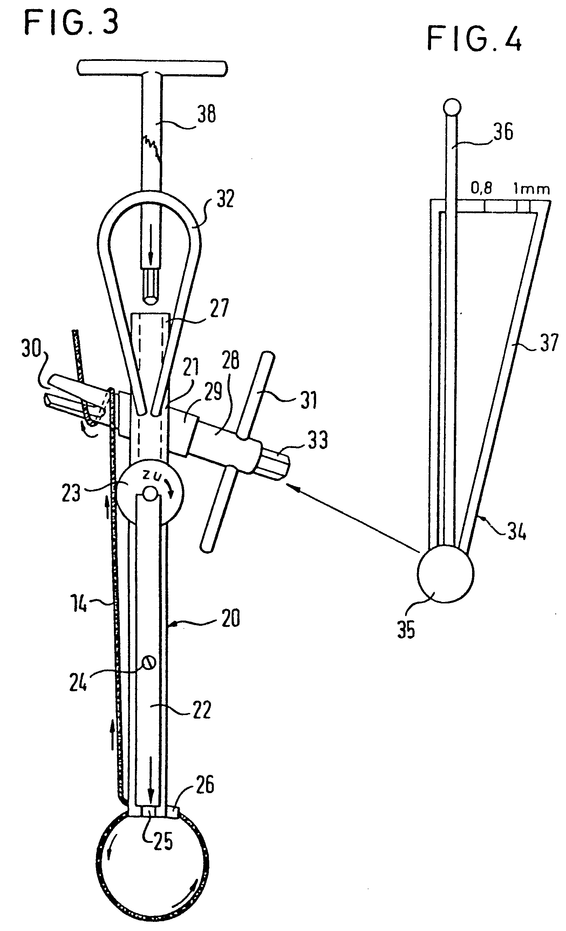

Die Spannspindel 28 hat an dem den Griff 31 aufweisenden Ende einen Sechskantzapfen, auf ein Drehmomentschlüssel 34 (Fig. 4) aufgesetzt wird.

Der Drehmomentschlüssel besteht aus einer Hülse 35 mit einer Sechskantbohrung, an der ein federnder Spannhebel 36 und ein Drehmomentanzeiger 37 befestigt sind.

Der vorgespannte Draht wird nun mittels des auf die Spannspindel 28 aufgesetzten Drehmomentschlüssels bis zur Fließgrenze gespannt. Hierzu wird der Spannhebel 36 in Fig. 4 nach rechts verstellt. Dabei entsteht eine Relativbewegung zwischen dem Spannhebel 36 und dem Drehmomentanzeiger 37. Sobald die Relativbewegung zwischen dem Spannhebel 36 und dem Drehmomentanzeiger 37 aufhört, d.h. die Fließgrenze des Drahtes erreicht ist und der Anzeiger sich von nun an zusammen mit dem Spannhebel 36 weiterbewegt, ist die höchste Belastbarkeit des Drahtes erreicht.

Der Spannvorgang wird nun dadurch beendet, daß mittels eines Inbusschlüssels 38, der in die Bohrung 27 des Spannhalters 20 eingesetzt ist, die Inbusschrabue 12 festgezogen und dadurch der Draht 14 in der Keilnut 16 festgeklemmt wird.

Nun wird der Draht zwischen dem Schloß und der Spannspindel 28 durchgetrennt. Durch entgegengesetzte Drehung der Mutter 23 wird der Hebel 22 entspannt und der Spannhalter kann vom Schloß abgezogen werden.

Im ungespannten Zustand des Drahtes verlaufen die beiden Drahtenden etwa parallel zueinander. Im gespannten Zustand erfolgt jedoch eine Drehung des Schlosses um etwa 45°, wie Fig. 2 zeigt. In dieser Stellung sind die aus dem Schloß vorstehenden Drahtteile abgebogen, so daß eine zusätzliche Spannwirkung erreicht wird. Damit der Draht an diesen Teilen nicht beschädigt wird, ist die Keilnut an den Enden abgerundet.

Eine weitere Spannwirkung wird dadurch erreicht, daß die Inbusschraube 12 beim Spannvorgang mit dem Gewindeteil unten über das Schloß vorsteht und sich am Knochen abstützt, wodurch das Schloß angehoben wird.

## Patentansprüche

1. Cerclageschloß zur Verwendung bei Knochenbrüchen, das zur Verbindung der Drahtenden eines um einen Knochen gespannten Drahtes dient, gekennzeichnet durch einen Vierkant-Blockkörper (11) mit einer Durchgangsbohrung (19) zur Aufnahme des einen mit einer Anschlagkugel (15) versehenen Drahtendes, eine Keilnut (16) zur Aufnahme des anderen Drahtendes, und eine durchgehende Gewindebohrung mit einer zylindrischen Ansatzbohrung zur Aufnahme einer Inbusschraube (12) mit einem Gewindeteil (13) und einem zylindrischen Kopf, der das andere Ende des Drahtes in die Keilnut (16) drückt.

2. Schloß nach Anspruch 1, dadurch gekennzeichnet, daß die Keilnut (16) an den Enden abgeschrägt ist.

3. Schloß nach Anspruch 1 oder 2, dadurch gekennzeichnet, daß der Gewindeteil (13) der Inbusschraube (12) im eingeschraubten Zustand etwas über die Unterseite des Schlosses vorsteht.

4. Schloß nach einem der Ansprüche 1 bis 3, dadurch gekennzeichnet, daß der Blockkörper (11) eine quadratische Grundfläche hat.

5. Schloß nach Anspruch 4, dadurch gekennzeichnet, daß die Durchgangsbohrung (19) und die Keilnut (16) längs jeweils einer Seite des Blockkörpers (11) parallel verlaufen.

6. Schloß nach Anspruch 4 oder 5, dadurch gekennzeichnet, daß die Durchgangsbohrung (19) von der Seite, an der die Anschlagkugel (15) des einen Drahtendes anliegt, schräg nach oben verläuft.

7. Schloß nach einem der Ansprüche 1 bis 6, dadurch gekennzeichnet, daß die Keilnut (16) sowohl nach unten als auch nach außen gewölbt ist.

8. Spannhalter zur Aufnahme des Schlosses nach einem der Ansprüche 1 bis 7, gekennzeichnet durch ein Rohr (21) mit einer durchgehenden Bohrung (27), die am unteren Ende in eine Vierkantöffnung (25) zur Aufnahme des Schlosses (11) übergeht, deren eine Seite als Spannbacken ausgebildet ist und mittels eines schwenkbaren Hebels (22) gegen das Schloß (11) gedrückt werden kann, eine Spannspindel (28) am oberen Ende des Rohrs (21), die in einer Hülse (29) drehbar und axial unverschiebbar gelagert ist und am einen Ende eine konische Nut (30) zur Aufnahme des einen, durch die Keilnut (16) des Schlosses geführten Drahtendes aufweist.

9. Spannhalter nach Anspruch 8, gekennzeichnet durch einen Drahtführungstrichter (26) am unteren Ende des Rohrs (21) und eine Ausnehmung neben dem Führungstrichter (26) zur Aufnahme des Anschlagkugel (15) am einen Drahtende.

10. Spannhalter nach Anspruch 8 oder 9, dadurch gekennzeichnet, daß der Hebel (22) mittels eines Schraube (24) befestigt und schwenkbar gelagert ist und mittels einer Rändelschraube (23) am oberen Ende des Hebels schwenkbar ist.

11. Spannhalter nach Anspruch 8, dadurch gekennzeichnet, daß die Nut (30) der Spindel (28) an der die Spannung des Drahtes bewirkenden Kante unter einem Winkel von 45° abgeschrägt ist.

12. Spannhalter nach Anspruch 8, gekennzeichnet durch einen Griff (31) am der Nut (30) entgegengesetzten Ende.

13. Spannhalter nach einem der Ansprüche 8 bis 12, gekennzeichnet durch einen seitlichen Griff (32).

14. Spannhalter nach einem der Ansprüche 8 bis 13, gekennzeichnet durch einen Sechskantzapfen (33) an dem der Nut (30) entgegengesetzten Ende der Spindel (28) zur Aufnahme eines Drehmomentschlüssels (34).

15. Spannhalter nach Anspruch 14, dadurch gekennzeichnet, daß der Drehmomentschlüssel (34) aus einer eine Sechskantbohrung aufweisenden Hülse (35), an der ein federnder Spannhebel (36) und ein Drehmomentanzeiger (37) befestigt sind, besteht.

16. Spannhalter nach einem der Ansprüche 8 bis 15, gekennzeichnet durch einen Inbusschlüssel (38) zum Festziehen der Inbusschraube (12) des Schlosses (11), der in die durchgehende Bohrung (27) des Rohrs (21) einsetzbar ist.

## Revendications

1. Serrure de cerclage à utiliser lors de fractures des os, et servant à relier les extrémités d'un fil tendu autour d'un os, caractérisée par un bloc à quatre pans (11) ayant un orifice traversant (19) destiné à recevoir l'une des extrémités de fil munie d'une bille de butée (15), par une rainure de coincement (16) destinée à recevoir l'autre extrémité du fil, et par un orifice taraudé traversant ayant un trou cylindrique à épaulement pour recevoir une vis à six pans creux (12) ayant une partie filetée (13) et une tête cylindrique qui pousse l'autre extrémité du fil dans la rainure de coincement (16).

2. Serrure selon la revendication 1, caractérisée en ce que la rainure de coincement (16) est biseautée aux extrémités.

3. Serrure selon la revendication 1 ou la revendication 2, caractérisé en ce que la partie filetée (13) de la vis à six pans creux (12) dépasse lorsqu'elle est vissée quelque peu au-dessus du côté inférieur de la serrure.

4. Serrure selon l'une des revendications 1 à 3, caractérisée en ce que le bloc (11) a une face de base carrée.

5. Serrure selon la revendication 4, caractérisée en ce que l'orifice traversant (19) et la rainure de coincement (16) s'étendent parallèlement chacun le long d'un côté du bloc (11).

6. Serrure selon la revendication 4 ou la revendication 5, caractérisée en ce que l'orifice traversant (19) s'étend obliquement vers le haut à partir du côté sur lequel repose la bille de butée (15) de l'une des extrémités de fil.

7. Serrure selon l'une des revendications 1 à 6, caractérisée en ce que la rainure de coincement (16) est courbée aussi bien vers le bas que vers l'extérieur.

8. Dispositif de maintien sous tension destiné à recevoir la serrure selon l'une des revendications 1 à 7, caractérisé par un tube (21) ayant un orifice traversant (27) qui passe à l'extrémité inférieure dans une ouverture à quatre pans (25) destinée à recevoir la serrure (11) dont l'un des côtés est réalisé comme une mâchoire de serrage et peut être poussé au moyen d'un levier pivotant (22) contre la serrure (11), par un arbre de serrage (28) à l'extrémité supérieure du tube (21), cet arbre étant monté de manière à pouvoir tourner dans une douille (29) et à ne pas pouvoir se déplacer axialement et présentant à une extrémité une rainure conique (30) destinée à recevoir l'une des extrémités de fil guidée par le rainure de coincement (16) de la serrure.

9. Dispositif selon la revendication 8, caractérisé par un bec de guidage de fil (27) à l'extrémité inférieure du tube (21) et un décrochement près du bec de guidage (26) pour recevoir la bille de butée (15) sur l'une des extrémités de fil.

10. Dispositif selon la revendication 8 ou 9, caractérisé en ce que le levier (22) est fixé au moyen d'une vis (24) et est monté de façon à pouvoir pivote et pivote au moyen d'une vis moletée (23) à l'extrémité supérieure du levier.

11. Dispositif selon la revendication 8, caractérisé en ce que la rainure (30) de l'arbre (28) est biseauté sur le bord occasionnant la tension du fil, sous un angle de 45°.

12. Dispositif selon la revendication 8, caractérisé par une poignée (31) du côté opposé à la rainure (30).

13. Dispositif selon l'une des revendications 8 à 12, caractérisé par une poignée latérale (32).

14. Dispositif selon l'une des revendications 8 à 13, caractérisé par une broche hexagonale (33) sur l'extrémité opposée à la rainure (30) de l'arbre (28) pour recevoir une clé à moment de rotation (34).

15. Dispositif selon la revendication 14, caractérisé en ce que la clé à moment de rotation (34) est constituée par une douille (35) présentant un orifice à six pans et sur laquelle sont fixés un levier de serrage (36) élastique et un indicateur de moment de rotation (37).

16. Dispositif selon l'une des revendications 8 à 15, caractérisé par une clé à six pans (38) pour serrer la vis à six pans (12) de la serrure (11) qui peut être introduite dans l'orifice traversant (27) du tube (21).

## Claims

1. Hooping lock for use in bone fractures, which acts to join the wire ends of a wire tightened around a bone, characterised by a square block element (11) having a bored passage (19) for reception of the one wire end provided with a stop ball (15), a tapered groove (16) for reception of the other wire end, and a transpiercing tapped bore comprising a cylindrical counterbore for reception of a hollow screw (12) comprising a screw-threaded section (13) and a cylindrical head which thrusts the other end of the wire into the tapered groove (16).

2. Lock according to claim 1, characterised in that the groove (16) is chamfered at its extremities.

3. Lock according to claim 1 or 2, characterised in that the screw-threaded part (13) of the hollow screw (12) projects a little beyond the underside of the lock in the screwed-in condition.

4. Lock according to one of the claims 1 to 3, characterised in that the block element (11) has a square base area.

5. Lock according to claim 4, characterised in that the bored passage (19) and the groove (16) extend parallel along one side of the block element (11) in each case.

6. Lock according to claim 4 or 5, characterised in that the bored passage (19) extends upwards obliquely from the side at which the stop ball (15) of the one wire end is in contact.

7. Lock according to one of the claims 1 to 6, characterised in that the groove (16) is outwardly as well as downwardly curved.

8. Tensioner for reception of the lock according to one of the claims 1 to 7, characterised by a tube (21) having a through-bore (27) which at the lower extremity merges into a square opening (25) for reception of the lock (11), one side of which is formed as a clamping jaw and may be pressed against the lock (11) by means of a pivotable lever (22), a tensioning spindle (28) at the upper extremity of the tube (21) which is fitted in a sleeve (29) in rotatable and axially fixed manner and at one extremity has a notch (30) for reception of the one wire end led through the groove (16) of the lock.

9. Tensioner according to claim 8, characterised by a wire guide funnel (26) at the lower extremity of the tube (21) and a recess beside the guide funnel (26) for reception of the stop ball (15) on the one wire end.

10. Tensioner according to claim 8 or 9, characterised in that the lever (22) is secured and pivotally mounted by means of a screw (24) and may be pivoted by means of a milled-edge screw (23) on the upper extremity of the lever.

11. Tensioner according to claim 8, characterised in that the notch (30) of the spindle (28) is chamfered at an angle of 45° at the edge establishing the tension of the wire.

12. Tensioner according to claim 8, characterised by a handle (31) at the other extremity from the notch (30).

13. Tensioner according to one of the claims 8 to 12, characterised by a lateral handle (32).

14. Tensioner according to one of the claims 8 to 13, characterised by a hexagonal shank (33) at the opposite extremity of the spindle (28) from the notch (30), for reception of a torque wrench (34).

15. Tensioner according to claim 14, characterised in that the torque wrench (34) comprises a sleeve (35) having a hexagonal bore and on which are fastened a springy tensioning lever (36) and a torque indicator (37).

16. Tensioner according to one of the claims 8 to 14, characterised by an Allen key (38) for tightening the hollow screw (12) of the lock (11), which is insertible into the through-bore (27) of the tube (21).

FIG.1

FIG.2

Knochen

FIG.3 FIG.4

2